# EUROPEAN PATENT APPLICATION

(11) **EP 2 730 512 A1**
(43) Date of publication of application: **14.05.2014**
(21) Application number: 12811422.0
(22) Date of filing: 05.07.2012
(51) Int. Cl.: B65B 55/10, A61L 2/10, A61L 2/20

(54) **METHOD FOR STERILIZING PACKAGING CONTAINER USING DILUTE HYDROGEN PEROXIDE-CONTAINING GAS**

(30) Priority: 08.07.2011 JP 2011151683
(71) Applicant: Shikoku Kakoki Co., Ltd., Itano-gun Tokushima 771-0287 (JP)
(72) Inventor: ITOH, Yasumasa, Itano-gun Tokushima 771-0287 (JP); NISHIO, Yoji, Itano-gun Tokushima 771-0287 (JP)
(74) Representative: Duckett, Anthony Joseph
(86) International application number: PCT/JP2012/004367
(87) International publication number: WO 2013/008426

(57) **Abstract**

A method for sterilizing a packaging container is provided which satisfies energy-saving requirements with consideration for energy cost and initial cost, which has high sterilization performance, and which is employable to make a highly safe dry sterilization system. The method for sterilizing a packaging container comprises: (a) a step of jetting a hydrogen peroxide-containing gas at a temperature of 10 to 60°C, preferably 10°C to 40°C, and at a concentration of 10 to 200 ppm (for example, an unsaturated hydrogen peroxide-containing gas that is obtained by supplying air into a gas-phase portion in a tank reserving hydrogen peroxide solution at a concentration of 3 to 40%) into a packaging container to be sterilized to bring hydrogen peroxide into contact with an inner surface of the packaging container; (b) a step of irradiating the inner surface of the packaging container with ultraviolet radiation; and (c) a purging step of jetting sterile air at a temperature of 10 to 60°C, preferably at room temperature, into the packaging container to replace the hydrogen peroxide-containing gas in the packaging container with the sterile air.

## Description

### Technical Field

The present invention relates to an energy-saving-type method and system for sterilizing a packaging container with a dilute hydrogen peroxide-containing gas.

### Background Art

Conventionally, for the purpose of extending the shelf life of liquid-food products such as milk and soft drinks, sterilization of paper containers and plastic containers with a sterilizing agent such as hydrogen peroxide prior to filling of the containers with the liquid-food products has widely been conducted. For example, a method which includes: a step of jetting a mist of hydrogen peroxide or a high-temperature gas of hydrogen peroxide onto a surface of a container to cause the hydrogen peroxide to condense on the surface; and a subsequent step of purging the hydrogen peroxide by dry removal with hot air is well known (see for example, Patent Document 1). Further, a method which involves combined use of hydrogen peroxide and ultraviolet irradiation for higher sterilizing power is known. For example, a method which includes treating a microorganism with an ultraviolet irradiated solution of hydrogen peroxide, the wavelength of the ultraviolet irradiation being wholly or predominantly below 325 nm and the concentration of the hydrogen peroxide being no greater than 10% by weight (see for example, Patent Document 2), a method which includes: a sterilizing agent attaching step of attaching a hydrogen peroxide-containing solution to an inner part of a food-packaging container having an opening in an upper part thereof; a UV sterilization step of irradiating, with ultraviolet radiation, the inner part of the container to which hydrogen peroxide was attached and thereby sterilizing the inner part of the container; and a sterilizing agent removing step of blowing an inert gas serving as a purge gas onto the inner part of the container to which the hydrogen peroxide was attached and thereby removing the hydrogen peroxide from the inner part of the container (see for example, Patent Document 3), and a method for producing a packaging container with very low microbiological load by treating a packaging material with a combination of hydrogen peroxide (H₂O₂) at a concentration in the range of 0.1 to 5% by weight and ultraviolet radiation (see for example, Patent Document 4) are known.

### Prior Art Documents

### Patent Documents

Patent Document 1: Japanese unexamined Patent Application Publication No. 2009-280222
Patent Document 2: Japanese unexamined Patent Application Publication (Translation of PCT Application) No. 56-500058
Patent Document 3: Japanese unexamined Patent Application Publication No. 2004-59014
Patent Document 4: Japanese unexamined Patent Application Publication (Translation of PCT Application) No. 2010-521378

### Summary of the Invention

### Object to be Solved by the Invention

In the case of ultraviolet irradiation after jetting of a mist of hydrogen peroxide, high-temperature air is required for quick dry removal of hydrogen peroxide attached to the container; moreover, in a case where the container has a complex shape, there are some parts in the container where the mist of hydrogen peroxide is not sufficiently attached, with the result that sterilization ends up in failure. Meanwhile, in the case of ultraviolet irradiation after jetting of hydrogen peroxide that has been turned into a high-temperature gas, it is necessary to heat hydrogen peroxide to its boiling point or higher, although the shortcoming of jetting of a mist of hydrogen peroxide is compensated. This makes it necessary to provide a heat source for the gasifying step in addition to providing a heat source for the drying step, thus incurring ballooning apparatus cost and running cost. An object of the present invention is to provide a method for sterilizing a packaging container which satisfies energy-saving requirements with consideration for energy cost and initial cost, which has high sterilization performance, and which is employable to make a highly safe dry sterilization system.

### Means to Solve the Object

The present inventors diligently studied to develop a sterilization system which satisfies energy-saving requirements while maintaining high sterilization performance. As a result, the present inventors reached the conclusion that in order to satisfy energy-saving requirements, it is essential to eliminate or reduce heating in the step of purging hydrogen peroxide having adhered to the inner surface of a container in a conventional hydrogen peroxide sterilization system. In view of this conclusion, the present inventors attempted various experiments on energy saving in the purging step, but failed to clear the problem of residual hydrogen peroxide. In face of this failure, the present inventors changed the way of thinking to work out a sterilization system which does not require heated gas for the purging step. For that purpose, the present inventors examined sterilization performance as exhibited in a case where a normal-temperature dilute hydrogen peroxide gas was used instead of a mist of hydrogen peroxide or a high-temperature gas of hydrogen peroxide. As a result, the present inventors obtained the following findings: a normal-temperature gas containing dilute hydrogen peroxide is obtained by reserving a hydrogen peroxide solution in a tank provided with an inlet and an outlet and by supplying air into a gas phase; a dilute hydrogen peroxide-containing gas is obtained more efficiently by using dry air as an air source; bubbling in a hydrogen peroxide solution increases the efficiency with which hydrogen peroxide is contained in a gas phase, but requires a complex system, and a large amount of air makes it easy for a fine mist to spread due to bubbling, thus increasing the risk of the mist adhering to the inner surface of a packaging container; sufficient sterilization performance cannot be attained by treatment with a dilute hydrogen peroxide-containing gas alone, and high sterilization performance can be attained with concomitant use of ultraviolet irradiation (that is, concomitant use of ultraviolet irradiation is indispensable); since this system does not cause hydrogen peroxide to be attached to or condense on the inner surface of a container, purging after ultraviolet irradiation is done without problems simply by blowing normal-temperature air in an amount that is equivalent to the volume of the carton, while a conventional system needs high-temperature air purging to avoid residual hydrogen peroxide after a mist of hydrogen peroxide or a high-temperature gas of hydrogen peroxide has been jetted; and the like. On the basis of these findings, the present inventors accomplished the present invention.

That is, the present invention relates to (1) a method for sterilizing a packaging container, the method comprising: (a) a step of jetting a hydrogen peroxide-containing gas at a temperature of 10 to 60°C and at a concentration of 10 to 200 ppm into a packaging container to be sterilized to bring hydrogen peroxide into contact with an inner surface of the packaging container; (b) a step of irradiating the inner surface of the packaging container with ultraviolet radiation; and (c) a purging step of jetting sterile air at a temperature of 10 to 60°C into the packaging container to replace the hydrogen peroxide-containing gas in the packaging container with the air, (2) the sterilization method according to (1), wherein the hydrogen peroxide-containing gas in step (a) is an unsaturated gas that is obtained by supplying air into a gas-phase portion in a tank reserving an aqueous hydrogen peroxide solution at a temperature of 10°C to 40°C and at a concentration of 3 to 40%, (3) the sterilization method according to (2), wherein the unsaturated gas is obtained by supplying the air at 50 to 200 L/min, and (4) the sterilization method according to any one of (1) to (3), wherein the sterile air at a temperature of 10 to 60°C in step (c) is sterile air at room temperature.

Further, the present invention relates to (5) a system for sterilizing a packaging container, the system comprising: a dilute hydrogen peroxide-containing gas jetting apparatus; an ultraviolet irradiation apparatus; and an apparatus for introducing sterile air for purging a hydrogen peroxide-containing gas, the dilute hydrogen peroxide-containing gas jetting apparatus including a reservoir tank for reserving a hydrogen peroxide solution, provided with an inlet and an outlet; an air supply pipe connected to the inlet of the reservoir tank in communication therewith; a compressor for pumping air through the air supply pipe into a gas-phase portion in the reservoir tank; a dilute hydrogen peroxide-containing gas exhaust pipe connected to the outlet of the reservoir tank in communication therewith; a hydrogen peroxide concentration measurement device for measuring a concentration of hydrogen peroxide in a dilute hydrogen peroxide-containing gas, provided in the exhaust pipe; and a jetting nozzle for jetting the dilute hydrogen peroxide-containing gas, provided at an end of the exhaust pipe; and (6) the sterilization system according to (5), wherein the dilute hydrogen peroxide-containing gas jetting apparatus includes a hydrogen peroxide solution temperature measurement device provided in a liquid-phase portion in the reservoir tank and/or a hydrogen peroxide-containing gas temperature measurement device provided in the dilute hydrogen peroxide-containing gas exhaust pipe.

### Effect of the Invention

The present invention makes it possible to provide a high-sterilization-performance, safe, and energy-saving type dry sterilization system with consideration for energy cost and initial cost. Further, a method for sterilizing a packaging container of the present invention has been proven to also bring about a sufficient sterilizing effect on ultraviolet-resistant fungi.

### Brief Description of Drawings

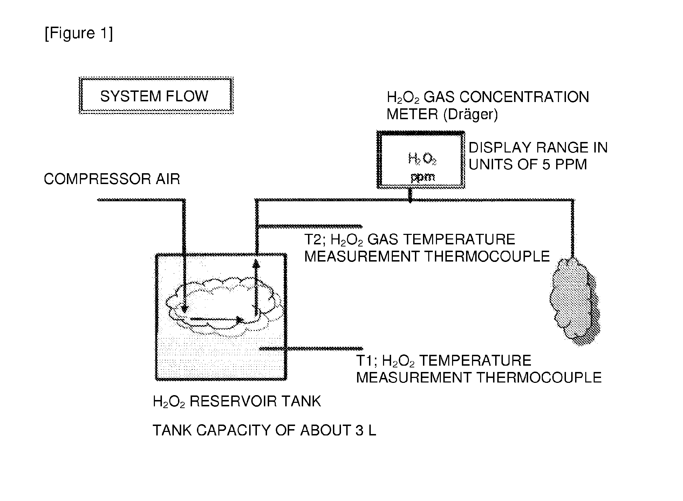

[Figure 1] Figure 1 is a diagram schematically showing a dilute hydrogen peroxide-containing gas jetting apparatus of a sterilization system based on combined use of a naturally-vapored hydrogen peroxide-containing gas and ultraviolet irradiation.

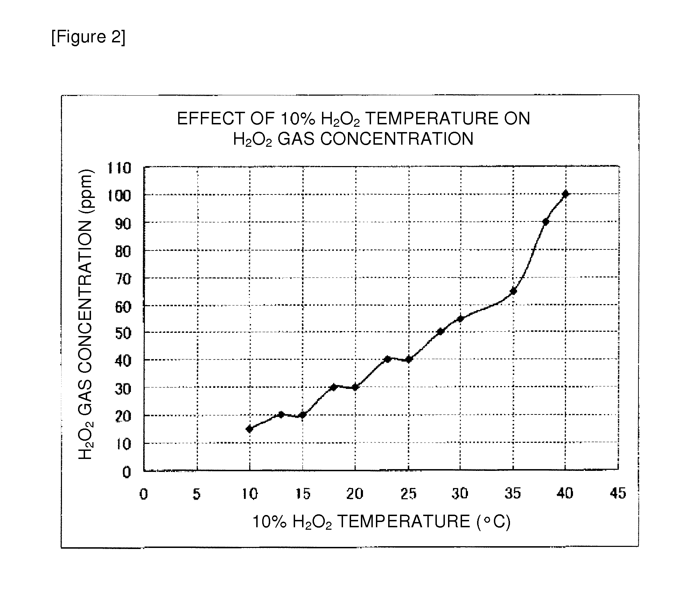

[Figure 2] Figure 2 is a graph showing the result of examination of the effect of the temperature of a hydrogen peroxide solution on the concentration of hydrogen peroxide in a hydrogen peroxide-containing gas.

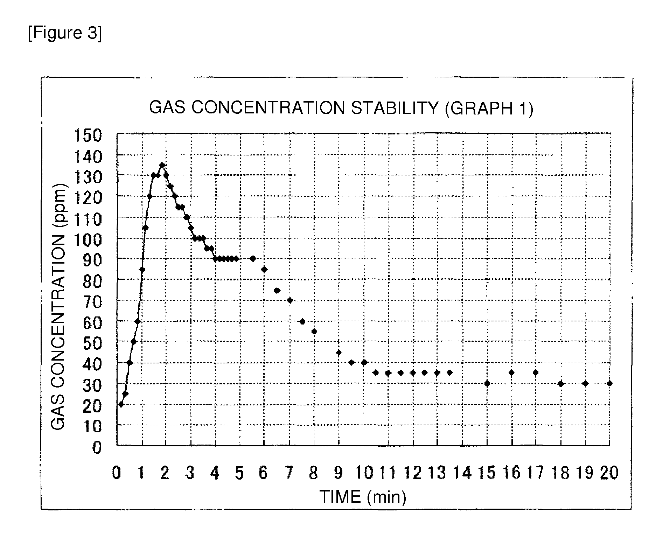

[Figure 3] Figure 3 is a graph showing the result of measurement of changes over time in the concentration of hydrogen peroxide in a hydrogen peroxide-containing gas at the start of supply of air, in the dilute hydrogen peroxide-containing gas jetting apparatus of the sterilization system of the present invention.

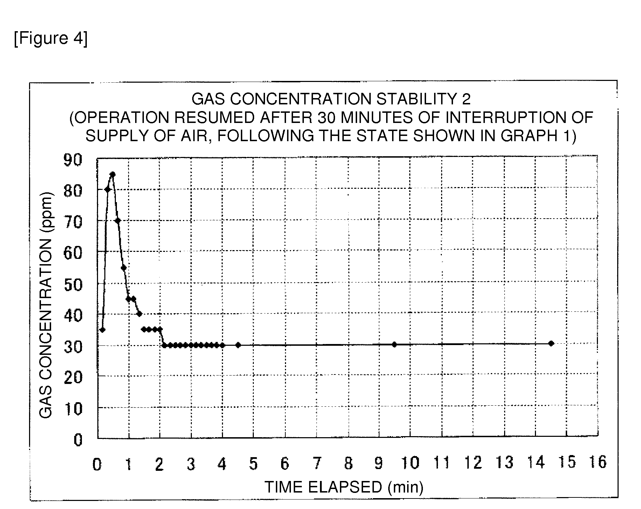

[Figure 4] Figure 4 is a graph showing the result of measurement of changes over time in the concentration of hydrogen peroxide in a hydrogen peroxide-containing gas after interruption of the supply of air, in the dilute hydrogen peroxide-containing gas jetting apparatus of the sterilization system of the present invention.

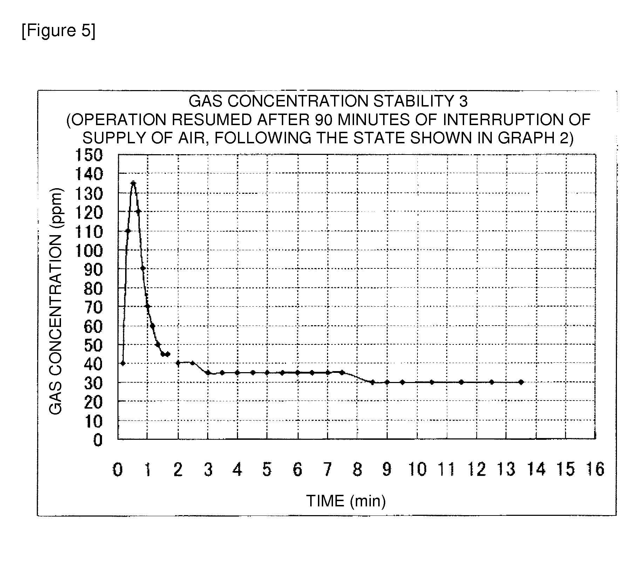

[Figure 5] Figure 5 is a graph showing the result of measurement of changes over time in the concentration of hydrogen peroxide in a hydrogen peroxide-containing gas after interruption of the supply of air, in the dilute hydrogen peroxide-containing gas jetting apparatus of the sterilization system of the present invention.

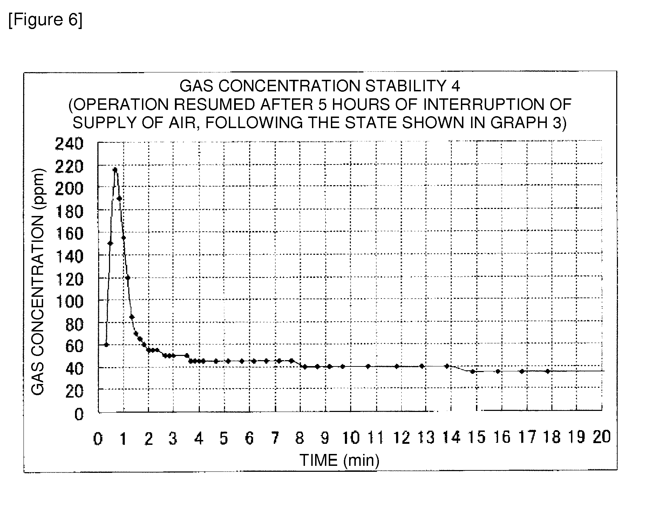

[Figure 6] Figure 6 is a graph showing the result of measurement of changes over time in the concentration of hydrogen peroxide in a hydrogen peroxide-containing gas after interruption of the supply of air, in the dilute hydrogen peroxide-containing gas jetting apparatus of the sterilization system of the present invention.

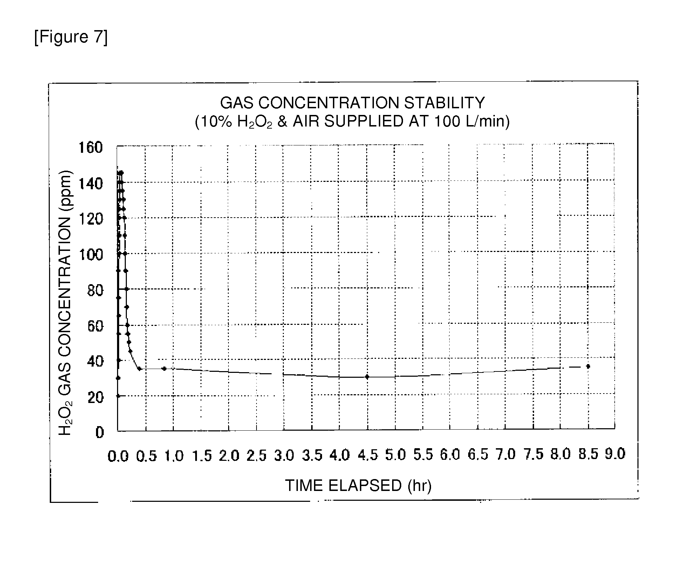

[Figure 7] Figure 7 is a graph showing the result of measurement of changes over time in the concentration of hydrogen peroxide in a hydrogen peroxide-containing gas at the start of supply of air, in the dilute hydrogen peroxide-containing gas jetting apparatus of the sterilization system of the present invention (the graph being obtained by performing the same measurement as that of GRAPH 1 for a longer period of time).

### Mode of Carrying Out the Invention

A method for sterilizing a packaging container of the present invention is not particularly limited, provided that it is a method comprising: (a) a step of jetting a hydrogen peroxide-containing gas at a temperature of 10 to 60°C and at a concentration of 10 to 200 ppm into a packaging container to be sterilized to bring hydrogen peroxide into contact with an inner surface of the packaging container; (b) a step of irradiating the inner surface of the packaging container with ultraviolet radiation; and (c) a purging step of jetting sterile air at a temperature of 10 to 60°C into the packaging container to replace the hydrogen peroxide-containing gas in the packaging container with the sterile air. Further, a system for sterilizing a packaging container of the present invention is not particularly limited, provided that it is a system comprising: a dilute hydrogen peroxide-containing gas jetting apparatus; an ultraviolet irradiation apparatus; and an apparatus for introducing sterile air for purging a hydrogen peroxide-containing gas, the dilute hydrogen peroxide-containing gas jetting apparatus including a reservoir tank for reserving a hydrogen peroxide solution, provided with an inlet and an outlet; an air supply pipe connected to the inlet of the reservoir tank in communication therewith; a compressor for pumping air through the air supply pipe into a gas-phase portion in the reservoir tank; a dilute hydrogen peroxide-containing gas exhaust pipe connected to the outlet of the reservoir tank in communication therewith; a hydrogen peroxide concentration measurement device for measuring a concentration of hydrogen peroxide in a dilute hydrogen peroxide-containing gas, provided in the exhaust pipe; and a jetting nozzle for jetting the dilute hydrogen peroxide-containing gas, provided at an end of the exhaust pipe. The packaging container is filled with a liquid-food product such as milk and juice.

The hydrogen peroxide-containing gas in step (a) of the method for sterilizing a packaging container of the present invention is preferably an unsaturated gas (unsaturated hydrogen peroxide gas) that is obtained by supplying air, preferably air at room temperature, at 50 to 200 L/min, preferably at 80 to 120 L/min, through a filter into a gas-phase portion in a tank reserving an aqueous hydrogen peroxide solution at a temperature of 10°C to 40°C, preferably at room temperature, and at a concentration of 3 to 40%, preferably at a concentration of 8 to 35%. The hydrogen peroxide-containing gas is jetted into the container, such as a carton, and makes contact with the inner surface of the container in such a state as not to be attached to or condense on the inner surface of the container.

Examples of the ultraviolet radiation in step (b) of the method for sterilizing a packaging container of the present invention include: short-wavelength ultraviolet radiation at a wavelength in the range of about 200 to about 280 nm, preferably at a wavelength of about 265 nm with sterilizing power close to the maximum sensitivity of a nucleic acid; medium-wavelength ultraviolet radiation at a wavelength in the range of about 280 to about 320 nm; and long-wavelength ultraviolet radiation at a wavelength in the range of about 320 to about 400 nm. Examples of ultraviolet irradiation include irradiation with pulsed light containing ultraviolet radiation, as well as ultraviolet irradiation by a low-pressure mercury lamp and ultraviolet irradiation by a high-pressure mercury lamp. Further, it is preferable that a reflective plate for ultraviolet irradiation be of a light-condensing type.

In the purging step (c) of the method for sterilizing a packaging container of the present invention, the hydrogen peroxide-containing gas in the container, such as a carton, is replaced with the sterile air for removal. Since, as mentioned above, the hydrogen peroxide-containing gas jetted into the container, such as a carton, in step (a) is not attached to or does not condense on the inner surface of the container, the sterile air for purging in step (c) of the method for sterilizing a packaging container of the present invention needs only be sterile air at a temperature of 10 to 60°C, particularly sterile air at room temperature. Further, the sterile air needs only be supplied in an amount that is equivalent to the volume of the container, such as a carton.

It is preferable that the dilute hydrogen peroxide-containing gas jetting apparatus of the system for sterilizing a packaging container of the present invention includes control means for controlling the concentration, the temperature, and the amount jetted of the dilute hydrogen peroxide-containing gas, as well as a hydrogen peroxide solution temperature measurement device provided in a liquid-phase portion in the reservoir tank, and a hydrogen peroxide gas concentration measurement device provided in the dilute hydrogen peroxide-containing gas exhaust pipe. A usable example of the hydrogen peroxide gas concentration measurement device includes a measuring instrument named Dräger Polytron 7000 (gas sensor; gas sensor for high-concentration hydrogen peroxide, with a measurement range of 0 to 7000 ppm, a display resolution of 5 ppm, and a minimum display of 5 ppm; manufactured by Drägerwerk AG & Co. KGaA). It is preferable that the control means has a mechanism which controls the amount of the sterile air for purging that is supplied.

The present invention is described below with reference to Examples, etc. Note, however, that the technical scope of the present invention is not to be limited by these Examples, etc.

### Examples

### [Example 1]

### [Preliminary Experiment]

Figure 1 schematically shows a sterilization system based on combined use of a naturally-vaporized hydrogen peroxide-containing gas and ultraviolet irradiation. The sterilization system includes: a 3 L reservoir tank for reserving a hydrogen peroxide solution, provided with an inlet and an outlet; a compressor which pumps air into a gas-phase portion in the reservoir tank; a hydrogen peroxide solution temperature measurement thermocouple (T1) provided in a liquid-phase portion in the reservoir tank; a hydrogen peroxide-containing gas temperature measurement thermocouple (T2) provided in a pipe between the reservoir tank and a jetting nozzle (closer to the reservoir tank than to the jetting nozzle); and a concentration-measuring instrument (manufactured by Drägerwerk AG & Co. KGaA) for measuring the concentration of hydrogen peroxide in a hydrogen peroxide-containing gas, provided in the pipe between the reservoir tank and the jetting nozzle (closer to the jetting nozzle than to the reservoir tank). The concentration-measuring instrument is as follows:
Name of the measuring instrument: Dräger Polytron 7000
Gas sensor: gas sensor for high-concentration hydrogen peroxide
Measurement range: 0 to 7000 ppm
Display resolution: 5 ppm
Minimum display: 5 ppm.
Note that the amount of a hydrogen peroxide solution at each concentration loaded into the reservoir tank was 1 L. The sterilization system was used to conduct the following various experiments.

First, the effect of the concentration of hydrogen peroxide and the amount of air supplied on the concentration of hydrogen peroxide gas was examined. The results are shown in Table 1. These results show that a higher concentration of hydrogen peroxide in the solution and a smaller amount of air supplied into the gas-phase portion in the reservoir tank yield a hydrogen peroxide-containing gas having a higher concentration. Moreover, these results show that a hydrogen peroxide-containing gas having a hydrogen peroxide gas concentration of 30 to 35 ppm is obtained by supplying air at 19°C at 100 mL/min into a gas-phase portion in a tank reserving a 10% hydrogen peroxide solution.

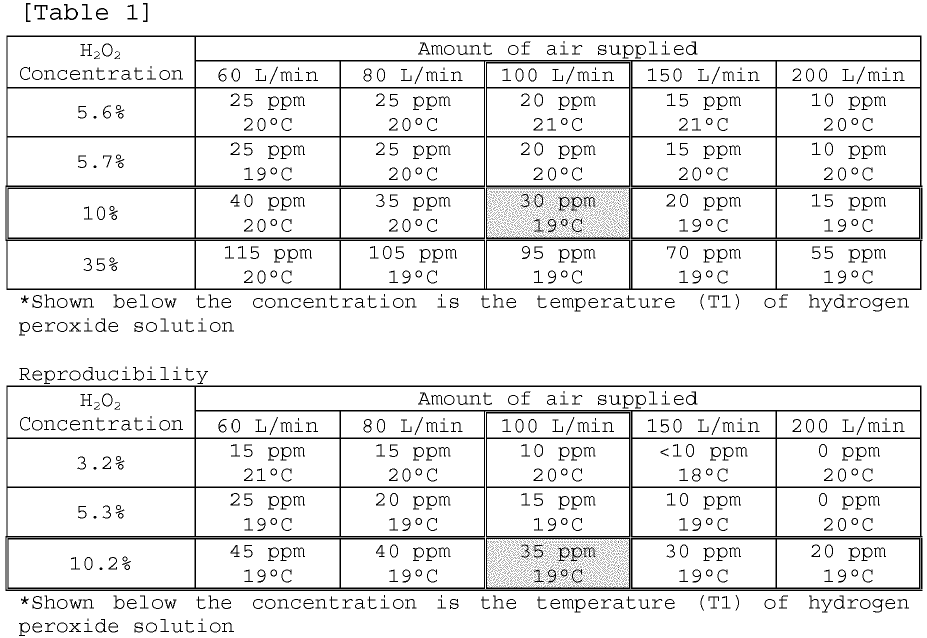

Next, the effect of the temperature (T1) of a hydrogen peroxide solution on the concentration of hydrogen peroxide in a hydrogen peroxide-containing gas was examined. Specifically, the gas concentration of a hydrogen peroxide-containing gas obtained by supplying air at 21.4°C at 100 mL/min into a gas-phase portion in a tank reserving a 10% hydrogen peroxide solution at differing temperatures was measured. The results are shown in Table 2. These results show that the higher the temperature of a hydrogen peroxide solution is, the higher the concentration of the resulting hydrogen peroxide-containing gas is. For example, these results show that when the temperature of a hydrogen peroxide solution is 20°C, the resulting hydrogen peroxide-containing gas has a hydrogen peroxide gas concentration of 35 ppm.

**[Table 2]**

| Amount of air supplied | Temperature of air supplied | Amount of H₂O₂ | Temperature of H₂O₂ | H₂O₂ gas concentration | Nozzle outlet temperature |
|---|---|---|---|---|---|
| 100 L/min | 21.4°C | 10% | 10°C | 20 ppm | |
| | | | 11°e | 20 ppm | |
| | | | 15°C | 25 ppm | |
| | | | 16°C | 30 ppm | |
| | | | 17°C | 35 ppm | |
| | | | 11°e | 15 to 20 ppm | 20.2°C |
| | | | 12°C | 20 ppm | |
| | | | 13°C | 20 ppm | |
| | | | 14°C | 25 ppm | |
| | | | 15°C | 25 ppm | 19.3°C |
| | | | 17°C | 25 to 30 ppm | |
| | | | 19°C | 35 ppm | 22.3°C |
| | | | 20°C | 35 ppm | |

Similar experiments were conducted on 10% and 35% hydrogen peroxide solutions. Table 3 (upper) shows the results of measurement of gas concentrations of hydrogen peroxide-containing gases. Further, the temperature (T1) of the 10% hydrogen peroxide solution was studied in detail. Figure 2 shows the results of measurement of gas concentrations of hydrogen peroxide-containing gases, and Table 3 (lower) shows the results together with the temperatures (T2) of hydrogen peroxide-containing gases. These results show that the higher the temperature of a hydrogen peroxide solution is, the higher the temperature and concentration of the resulting hydrogen peroxide-containing gas are.

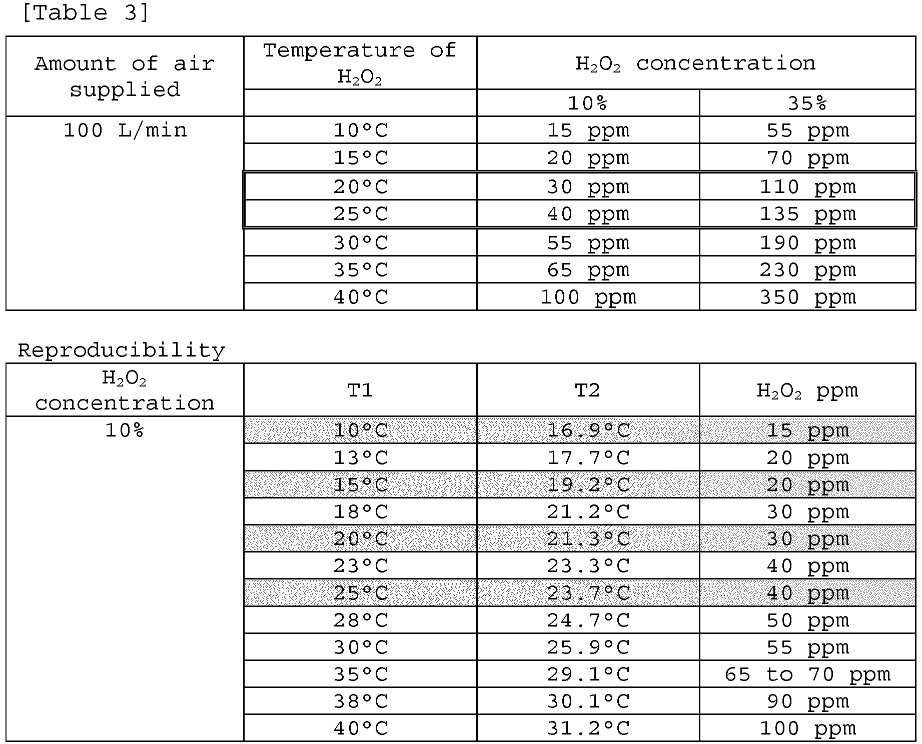

A package-filling machine provided with this type of sterilization system requires cleaning and sterilization in each case of occurrence of trouble, as well as daily cleaning and sterilization. In an actual case of application of a sterilization system of the present invention to a package-filling machine, the major issue is the gas concentration stability of a hydrogen peroxide-containing gas. With this in mind, gas concentration stability was assessed by examining changes over time in the gas concentration of a hydrogen peroxide-containing gas at the start of supply of and after interruption of supply of room-temperature air at 100 mL/min into a gas-phase portion in a tank reserving a 10% hydrogen peroxide solution at a temperature of 20°C. The results are shown in Figures 3 to 7. These results have confirmed that there is no actual operating problem with the gas concentration stability of a hydrogen peroxide-containing gas in a sterilization system of the present invention.

### [Example 2]

### [Evaluation of Carton Sterilization Performance]

Under the conditions indicated below, sterilization was carried out with combined use of a hydrogen peroxide-containing gas and ultraviolet irradiation. The indicator organisms for sterilization were spores of *Bacillus subtilis* or spores of *Aspergillus niger*, and 10⁶ of these spores of *Bacillus subtilis* or spores of *Aspergillus niger* were attached to the inner surface of each separate carton.
Duration of contact: 1 second
Duration of UV irradiation: 2.5 seconds (low-pressure UV); 1.0 second (high-pressure UV)
UV irradiator: 1.6 kW high-pressure mercury sterilization apparatus with three air-cooled 100 W lamps; manufactured by Iwasaki Electric Co., Ltd.
Gas jet: "Two nozzles", in the case of a carton fed by two pitches.

Purge nozzle: Inside diameter of 23 mm, purging with normal-temperature air at an airflow rate of 200 L/min.

Tables 4 to 9 show the results of evaluation of sterilization performance on the inner surfaces of cartons. Table 10 shows the results of evaluation of sterilization performance in the mouths of stoppered cartons. Table 11 shows a relationship between the concentration of hydrogen peroxide and the kill rate. These results have confirmed that on the inner surfaces of cartons in sizes of 1 L, 500 mL, and 250 mL and in the mouths of stoppered cartons, kill rates of about 3 to 4 (at which the number of organisms is 1/10³ to 1/10⁴) or higher can be attained by appropriately setting conditions such as the concentration of hydrogen peroxide, and that a sterilization system of the present invention has high sterilization performance.

**[Table 4]**

| Indicator organisms for sterilization | Carton size | UV lamp | Gas nozzle diameter | Amount of air supplied | H₂O₂ gas concentration | Kill rate |
|---|---|---|---|---|---|---|
| *B. subtilis* | 1 L | Three 100 W low-pressure UV lamps | Inside diameter of 10 mm | 110 L/min/ two nozzles | 55 to 60 ppm | 4.0 |
| | | | | | | 3.8 |
| | | | | | | 3.8 |
| | | | | | | 3.9 |
| | | | | 150 L/min/ two nozzles | 55 to 60 ppm | 3.7 |
| | | | | | | 4.1 |
| | | | | | | 3.8 |
| | | | | | | 3.8 |
| | | | | 200 L/min/ two nozzles | 55 to 60 ppm | 4.3 |
| | | | | | | 3.9 |
| | | | | | | 3.7 |
| | | | | | | 4.1 |
| | | | Inside diameter of 18.7 mm | 200 L/min/ two nozzles | 55 to 60 ppm | 3.8 |
| | | | | | | 4.8 |
| | | | | | | 3.9 |
| | | | | | | 4.2 |
| | | | | 250 L/min/ two nozzles | 55 to 60 ppm | 4.3 |
| | | | | | | 3.2 |
| | | | Inside diameter of 10 mm | 200 L/min/ two nozzles | 55 ppm | 3.5 |
| | | | | | | 4.6 |
| | | | | | | 3.8 |
| | | | | | | 4.3 |
| | | | | 150 L/min/ two nozzles | 20 to 25 ppm | 4.1 |
| | | | | | | 4.2 |
| | | | | | | 4.4 |
| | | | | | | 3.7 |
| | | | | 100 L/min/ two nozzles | 25 to 30 ppm | 3.9 |
| | | | | | | 4.2 |
| | | | | | | 4.3 |
| | | | Inside diameter of 18.7 mm | 150 L/min/ two nozzles | 60 ppm | 3.6 |
| | | | | | | 3.7 |
| | | | | | | 4.6 |
| | | | | | | 4.6 |
| | | | | 100 L/min/ two nozzles | 60 ppm | 4.4 |
| | | | | | | 3.9 |

**[Table 5]**

| Indicator organisms for sterilization | Carton size | UV lamp | Gas nozzle diameter | Amount of air supplied | H₂O₂ gas concentration | Kill rate |
|---|---|---|---|---|---|---|
| *B. subtilis* | 500 ml | Three 100 W low-pressure UV lamps | Inside diameter of 10 mm | 100 L/m two nozzles | 30 ppm | 4.4 |
| | | | | | | 4.3 |
| | | | | | | 4.4 |
| | | | | | | 4.9 |
| | | | | 150 L/m two nozzles | 30 ppm | 3.9 |
| | | | | | | 4.1 |
| | | | | | | 4.3 |
| | | | | | | 4.1 |
| | | | | 200 L/m two nozzles | 30 ppm | 3.8 |
| | | | | | | 4.6 |
| | | | | | | 4.4 |
| | | | | | | 4.0 |
| | 250 ml | Three 100 W low-pressure UV lamps | Inside diameter of 10 mm | 100 L/m two nozzles | 30 ppm | 4.6 |
| | | | | | | 4.3 |
| | | | | | | 4.9 |
| | | | | | | 4.4 |
| | | | | 150 L/m two nozzles | 30 ppm | 4.6 |
| | | | | | | 4.6 |
| | | | | | | 4.0 |
| | | | | | | 4.2 |
| | | | | 200 L/m two nozzles | 25 ppm | 4.2 |
| | | | | | | 4.3 |
| | | | | | | 4.0 |
| | | | | | | 3.8 |

**[Table 6]**

| Indicator organisms for sterilization | Carton size | UV lamp | Gas nozzle diameter | Amount of air supplied | H₂O₂ gas concentration | Kill rate |
|---|---|---|---|---|---|---|
| *B. subtilis* | 1 L | Three 100 W low-pressure UV lamps | Inside diameter of 10 mm | 80 L/min/ two nozzles | 30 ppm | 4.2 |
| | | | | | | 4.2 |
| | | | | | | 3.6 |
| | | | | | | 4.3 |
| | | | | 80 L/min/ two nozzles | 20 ppm | 3.9 |
| | | | | | | 4.1 |
| | | | | | | 3.7 |
| | | | | | | 4.0 |
| | | | | 80 L/min/ two nozzles | 15 ppm | 4.0 |
| | | | | | | 3.9 |
| | | | | | | 3.8 |
| | | | | | | 4.0 |
| | 250 ml | Three 100 W low-pressure UV lamps | Inside diameter of 10 mm | 100 L/min/ two nozzles | 30 ppm | 3.8 |
| | | | | | | 4.2 |
| | | | | | | 3.7 |
| | | | | | | 3.9 |
| | | | | 100 L/min/ two nozzles | 20 ppm | 3.7 |
| | | | | | | 4.2 |
| | | | | | | 3.7 |
| | | | | | | 3.8 |
| | | | | 100 L/min/ two nozzles | 15 ppm | 3.6 |
| | | | | | | 3.6 |
| | | | | | | 3.7 |

**[Table 7]**

| Indicator organisms for sterilization | Carton size | UV lamp | Gas nozzle diameter | Amount of air supplied | H₂O₂ gas concentration | Kill rate |
|---|---|---|---|---|---|---|
| *B. subtilis* | 1 L | Three 100 W low-pressure lamps | Inside diameter of 10 mm | 100 L/m two nozzles | 10 ppm | 3.0 |
| | | | | | | 3.0 |
| | | | | | | 2.9 |
| | | | | | | 3.2 |
| | | | | | 15 ppm | 3.6 |
| | | | | | | 3.9 |
| | | | | | | 3.7 |
| | | | | | | 3.8 |
| *A. niger* | 1 L | Three 100 W low-pressure lamps | Inside diameter of 10 mm | 100 L/m two nozzles | 100 ppm | 3.0 |
| | | | | | | 3.0 |
| | | | | | | 3.0 |
| | | | | | | 3.0 |
| | | | | | 60 ppm | 3.0 |
| | | | | | | 3.0 |
| | | | | | | 3.0 |
| | | | | | | 3.0 |
| | | | | | 30 ppm | 3.0 |
| | | | | | | 3.0 |
| | | | | | | 3.0 |

**[Table 8]**

| Indicator organisms for sterilization | Carton size | UV lamp | Gas nozzle diameter | Amount of air supplied | H₂O₂ gas concentration | Kill rate |
|---|---|---|---|---|---|---|
| *B. subtilis* | 1 L | High-pressure (1.6 KW) | Inside diameter of 10 mm | 100 L/m two nozzles | 15 ppm | 5.4 |
| | | | | | | 5.4 |
| | | | | | | 4.9 |
| | | | | | 30 ppm | >5.7 |
| | | | | | | >5.7 |
| | | | | | | >5.7 |
| | | | | | | >5.7 |
| | | | | | 50 ppm | 5.2 |
| | | | | | | 5.4 |
| | | | | | | >5.7 |
| | | | | | | 5.2 |
| | | | | | 80 ppm | 5.4 |
| | | | | | | 4.6 |
| | | | | | | 4.9 |
| | | | | | | 5.2 |

**[Table 9]**

| Indicator organisms for sterilization | Carton size | UV lamp | Gas nozzle diameter | Amount of air supplied | H₂O₂ gas concentration | Kill rate |
|---|---|---|---|---|---|---|
| *A. niger* | 1 L | High-pressure (1.6 KW) | Inside diameter of 10 mm | 100 L/m two nozzles | 15 ppm | 2.4 |
| | | | | | | 2.6 |
| | | | | | | 2.6 |
| | | | | | | 2.2 |
| | | | | | 30 ppm | 3.1 |
| | | | | | | 2.9 |
| | | | | | | 2.4 |
| | | | | | 50 ppm | >3.4 |
| | | | | | | >3.4 |
| | | | | | | 3.4 |
| | | | | | | 3.4 |
| | | | | | 100 ppm | >3.4 |
| | | | | | | 3.1 |
| | | | | | | 3.4 |
| | | | | | | 3.4 |

**[Table 10]**

| Indicator organisms for sterilization | Carton size | UV lamp | Gas nozzle diameter | Amount of air supplied | H₂O₂ gas concentration | Kill rate |
|---|---|---|---|---|---|---|
| *B. subtilis* | 1 L Stoppered | Three 100 W low-pressure lamps | Inside diameter of 10 mm | 100 L/min/ two nozzles | 15 ppm | 2.6 |
| | | | | | | 2.7 |
| | | | | | | 2.6 |
| | | | | | | 3.0 |
| | | | | | 30 ppm | 2.9 |
| | | | | | | 2.9 |
| | | | | | | 3.6 |
| | | | | | | 3.1 |
| | | | | | 50 ppm | 4.0 |
| | | | | | | 3.7 |
| | | | | | | 3.8 |
| | | | | | | 3.2 |
| | | | | | 100 ppm | 2.7 |
| | | | | | | 3.2 |
| | | | | | | 4.0 |
| | | | | | | 3.8 |

**[Table 11]**

| Indicator organisms for sterilization | Carton size | UV lamp | Gas nozzle diameter | Amount of air supplied | H₂O₂ gas concentration | H₂O₂ gas concentration | Kill rate |
|---|---|---|---|---|---|---|---|
| *B. subtilis* | 1 L | Three 100 W low-pressure lamps | Inside diameter of 10 mm | 100 L/min/ two nozzles | 5% | 15 ppm | 4.2 |
| | | | | | | | 4.3 |
| | | | | | | | 3.9 |
| | | | | | | | 4.0 |
| | | | | | 7% | 25 ppm | 3.9 |
| | | | | | | | 4.0 |
| | | | | | | | 4.0 |
| | | | | | | | 3.5 |
| | | | | | 10% | 30 ppm | 4.3 |
| | | | | | | | 4.2 |
| | | | | | | | 3.6 |
| | | | | | | | 4.1 |
| | | | | | 20% | 55 ppm | 3.9 |
| | | | | | | | 4.5 |
| | | | | | | | 4.2 |
| | | | | | | | 4.2 |
| | | | | | 35% | 100 ppm | 4.1 |
| | | | | | | | 4.1 |
| | | | | | | | 4.9 |
| | | | | | | | 4.9 |

### [Example 3]

### [Measurement of Residual Hydrogen Peroxide]

Residual concentrations of hydrogen peroxide were measured by conducting the following experiment: High-concentration hydrogen peroxide gases were each forcibly generated by warming (in hot water) a 3 L pressurized tank containing a 35% hydrogen peroxide solution, and each of the gases was jetted for 2 seconds, held for 3.6 seconds, and then purged for 1.2 seconds with normal-temperature air jetted at a wind velocity of 11 m/sec out of a purge nozzle having an inside diameter of 23 mm. The results are shown in Table 12. These results show that even in the case of purging with sterile air at normal temperature, the residual amounts of high-concentration hydrogen peroxide were within the acceptable range.

**[Table 12]**

| Carton size | Atomizing nozzle diameter | Amount of air supplied (amount of gas-atomized air) | Gas concentration (ppm) | Residual amount (ppm) |
|---|---|---|---|---|
| 1 L | Diameter 10 | 100 L/min/ two nozzles | 820 | 0.01 |
| | | | 880 | 0.00 |
| | | | 910 | 0.00 |
| | | 150 L/min/ two nozzles | 890 | 0.01 |
| | | | 935 | 0.01 |
| | | | 920 | 0.01 |
| | | 200 L/min/ two nozzles | 860 | 0.01 |
| | | | 900 | 0.01 |
| | | | 830 | 0.00 |
| 500 ml | Diameter 10 | 100 L/min/ two nozzles | 930 | 0.01 |
| | | | 920 | 0.01 |
| | | | 935 | 0.02 |
| | | 150 L/min/ two nozzles | 1100 | 0.01 |
| | | | 955 | 0.01 |
| | | | 880 | 0.01 |
| | | 200 L/min/ two nozzles | 810 | 0.02 |
| | | | 810 | 0.02 |
| | | | 770 | 0.01 |

Further, residual amounts of hydrogen peroxide resulting from super-harsh gas concentrations under the conditions indicated below were measured. 200 mL of a 35% aqueous hydrogen peroxide solution were loaded into a tank, and then warmed to 50°C with the tank in a water bath. Air regulated to 40°C by a heater was supplied at 100 L/min out of two nozzles per unit. With each gas jetting nozzle having an inside diameter of 10 mm, the resulting hydrogen peroxide-containing gas had a temperature of 32.0°C. Further, residual concentrations of hydrogen peroxide were measured by conducting the following experiment: each of the gases thus obtained was jetted for 1.2 seconds, held for 3.6 seconds, and then purged under the purge condition for 1.2 seconds with sterile air at an air temperature of 19.9°C jetted at an airflow rate of 200 L/min/nozzle out of a purge nozzle having an inside diameter of 23 mm. The results are shown in Table 13. These results show that the high-concentration gases, which were forcibly generated by warming, showed a trace of condensation, but even in the case of purging with sterile air at normal temperature, the residual amounts of high-concentration hydrogen peroxide were within the acceptable range. Meanwhile, once condensation occurs, a residual concentration cannot be cleared without purging.

**[Table 13]**

| Carton size | Atomizing nozzle diameter | Amount of air supplied (amount of gas-atomized air) | Gas concentration (ppm) | Residual amount (ppm) | |
|---|---|---|---|---|---|
| 1 L | Diameter 10 | 100 L/min/ two nozzles | 820 | 0.45 | *without purging |
| | | | 840 | 0.02 | |
| | | | 865 | 0.02 | |
| | | | 905 | 0.02 | |
| | | | 925 | 0.02 | |

### [Example 4]

### [Effect on the Amount of Evaporation by the Amount of Aqueous Hydrogen Peroxide Solution Loaded into a Tank]

Given amounts of an aqueous hydrogen peroxide solution were each loaded into a tank. The amount of air supplied was 100 L/min. The amounts of hydrogen peroxide solution decreased by evaporation were measured. The results are shown in Table 14. These results show that regardless of the amounts of an aqueous hydrogen peroxide solution that were each loaded into a tank, the hydrogen peroxide evaporates in the amount range of 20 to 25 g per hour.

**[Table 14]**

| Amount loaded into tank (g) | H₂O₂ % at start | Time elapsed (H) | Remaining amount (g) | H₂O₂ % at end | Amount of decrease (g/H) |
|---|---|---|---|---|---|
| 501.5 | 10.2 | 10 | 310.16 | 15.6 | 19.1 |
| 1006.1 | 10.2 | 8.5 | 809.1 | 12.4 | 23.2 |
| 1013.2 | 10 | 8.5 | 840.6 | 11.8 | 20.3 |
| 1013.2 | 9.8 | 8.5 | 840.6 | 11.6 | 20.3 |
| 1048.3 | 10.2 | 8.8 | 840 | 12.4 | 23.7 |
| 2104 | 10.2 | 9.5 | 1857.8 | 11.5 | 25.9 |

## Claims

1. A method for sterilizing a packaging container, the method comprising the following steps (a) to (c):
(a) a step of jetting a hydrogen peroxide-containing gas at a temperature of 10 to 60°C and at a concentration of 10 to 200 ppm into a packaging container to be sterilized to bring hydrogen peroxide into contact with an inner surface of the packaging container;
(b) a step of irradiating the inner surface of the packaging container with ultraviolet radiation; and
(c) a purging step of jetting sterile air at a temperature of 10 to 60°C into the packaging container to replace the hydrogen peroxide-containing gas in the packaging container with the air.

2. The sterilization method according to claim 1, wherein the hydrogen peroxide-containing gas in step (a) is an unsaturated gas that is obtained by supplying air into a gas-phase portion in a tank reserving an aqueous hydrogen peroxide solution at a temperature of 10°C to 40°C and at a concentration of 3 to 40%.

3. The sterilization method according to claim 2, wherein the unsaturated gas is obtained by supplying the air at 50 to 200 L/min.

4. The sterilization method according to any one of claims 1 to 3, wherein the sterile air at a temperature of 10 to 60°C in step (c) is sterile air at room temperature.

5. A system for sterilizing a packaging container, the system comprising:
a dilute hydrogen peroxide-containing gas jetting apparatus;
an ultraviolet irradiation apparatus; and
an apparatus for introducing sterile air for purging a hydrogen peroxide-containing gas,
the dilute hydrogen peroxide-containing gas jetting apparatus including a reservoir tank for reserving a hydrogen peroxide solution, provided with an inlet and an outlet; an air supply pipe connected to the inlet of the reservoir tank in communication therewith; a compressor for pumping air through the air supply pipe into a gas-phase portion in the reservoir tank; a dilute hydrogen peroxide-containing gas exhaust pipe connected to the outlet of the reservoir tank in communication therewith; a hydrogen peroxide concentration measurement device for measuring a concentration of hydrogen peroxide in a dilute hydrogen peroxide-containing gas, provided in the exhaust pipe; and a jetting nozzle for jetting the dilute hydrogen peroxide-containing gas, provided at an end of the exhaust pipe.

6. The system according to claim 5, wherein the dilute hydrogen peroxide-containing gas jetting apparatus includes a hydrogen peroxide solution temperature measurement device provided in a liquid-phase portion in the reservoir tank and/or a hydrogen peroxide-containing gas temperature measurement device provided in the dilute hydrogen peroxide-containing gas exhaust pipe.
